(19) Europäisches Patentamt
European Patent Office
Office européen des brevets

(11) Veröffentlichungsnummer: **0 063 538**
**B1**

(12) **EUROPÄISCHE PATENTSCHRIFT**

(45) Veröffentlichungstag der Patentschrift: **06.08.86**

(21) Anmeldenummer: **82730044.3**

(22) Anmeldetag: **06.04.82**

(51) Int. Cl.⁴: **C 07 D 521/00**, C 07 F 7/18, A 61 K 31/395 // C07C177/00, A61K31/557, (C07D521/00, 263:14, 277:10, 403:12, 413:08, 413:12, 417:12)

(54) **Neue Prostaglandine und Prostacycline, Verfahren zu ihrer Herstellung und ihre Verwendung als Arzneimittel.**

(30) Priorität: **13.04.81 DE 3115997**
**13.11.81 DE 3145830**

(43) Veröffentlichungstag der Anmeldung:
**27.10.82 Patentblatt 82/43**

(45) Bekanntmachung des Hinweises auf die Patenterteilung:
**06.08.86 Patentblatt 86/32**

(84) Benannte Vertragsstaaten:
**AT BE CH DE FR GB IT LI LU NL SE**

(56) Entgegenhaltungen:
**EP - A - 0 015 227**
**EP - A - 0 029 341**
**EP - A - 0 034 778**
**DE - A - 3 047 759**

**ANGEWANDTE CHEMIE, Band 19, Nr. 10, Oktober 1980, Seiten 749-840, H. KÖNIG: "Pharmaceutical chemistry today-changes, problems, and opportunities"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Vorbrüggen, Helmut, Prof., Wilkestrasse 7, D-1000 Berlin 27 (DE)**
Erfinder: **Elger, Walter, Dr., Schorlemer Allee 12b, D-1000 Berlin 33 (DE)**
Erfinder: **Town, Michael-Harold, Dr., Buchsbaumweg 3, D-1000 Berlin 47 (DE)**
Erfinder: **Schillinger, Ekkehard, Dr., Im Amseltal 50, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Prostaglandine und Prostacycline, Verfahren zu ihrer Herstellung sowie ihre Verwendung als Arzneimittel.

Als biologische Umwandlungsprodukte von drei-, vier- (Arachidonsäure) und fünffach ungesättigten natürlich vorkommenden $C_{20}$-Fettsäuren sind vor allem Prostaglandine, Prostacycline und Thromboxane, insbesondere in Form ihrer Analoga, von grosser therapeutischer Bedeutung.

Für die Wechselwirkung dieser Substanzen mit dem Rezeptor, d.h. für die Selektivität und Dauer der biologischen Wirkung sowie für den Stoffwechsel (β-Oxydation) ist die Funktionalität der 1-Carboxylgruppe von grosser Wichtigkeit. Verglichen mit den 1-Carbonsäuren haben 1-Ester, 1-Amide und insbesondere 1-Sulfon- oder 1-Acylamide ein verändertes biologisches Wirkungsspektrum [vgl. u.a. T.K. Schaaf und H.J. Hess, J. Med. Chem. 22, 1340 (1979)].

Aber alle diese Gruppen sind wie die 1-Ester oder Amide entweder neutral oder wie die 1-Acylsulfonamide sauer. Deshalb war es von grossem Interesse, die 1-Carboxylgruppe in Prostaglandinen, Prostacyclinen und Thromboxanen sowie ihrer Analoga ohne ihre Wertigkeit zu verändern, in basische Derivate zu überführen.

In EP-A-15 227, -29 341 und -34 778 sowie in Angew. Chem. 19, 90:820 (1980) werden Azaprostacycline beschrieben.

Mit Hilfe einer neuen in der DE-A-3 047 759 beschriebenen Methode ist es nun leicht möglich, die 1-Carboxylgruppe dieser Azaprostacycline und anderer Prostaglandine und Prostacycline in die entsprechenden basischen $\Delta^2$-Ozazoline, $\Delta^2$-Thiazoline bzw. $\Delta^2$-Imidazoline sowie ihrer höher gliedrigen Analoga wie z.B. 5,6-Dihydro-4H-1,3-oxazine, 5,6-Dihydro-4H-1,3-thiazine und Tetrahydropyrimidine zu überführen.

Diese neuen Derivate zeigen ein verändertes und selektiveres biologisches Wirkungsspektrum und sind insbesondere im Falle der Prostacycline chemisch und metabolisch bedeutend stabiler und daher länger wirksam als Prostacyclin ($PGI_2$).

Die Erfindung betrifft Prostaglandine der allgemeinen Formel I,

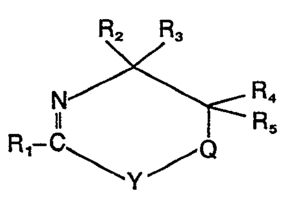

I,

in der

$R_1$ einen Prostaglandinrest der Formel

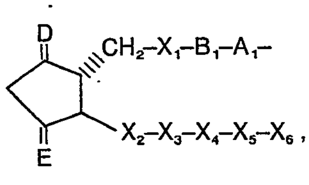

wobei

$A_1$ für $-(CH_2)_n-$, $-(CH_2)_m-O-$, $-CF_2-(CH_2)_m-$ mit n = 1–3 und m = 1–2 und $-CH=CH-CH_2-$, $-CH_2-CH=CH-$ und

$B_1-X_1$ für $-(CH_2)_3-$ oder $-CH=C=CH-$,

$A_1-B_1-X_1$ für $-(CH_2)_5-$,

$A_1-B_1$ für $-(CH_2)_o-$, $-(CH_2)_n-O-$, $-CF_2-(CH_2)_n-$, $-CH=CH-(CH_2)_m-$, $-(CH_2)_m-CH=CH-$ mit m = 1–2, n = 1–3 und o = 1–4 und

$X_1$ für ein cis-Alkenylen

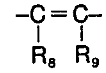

mit $R_8$ und $R_9$ als Wasserstoff oder $C_{1-C6}$-Alkyl sowie

$A_1$ für $-CH_2-$,

$B_1$ für Sauerstoff oder $-CH_2-$ und

D für Sauerstoff, Wasserstoff und α- oder β-Hydroxy, Wasserstoff und α- oder β-Halogen oder $CH_2$,

E für Sauerstoff, Wasserstoff und α-Hydroxy, Wasserstoff und α-$CH_3$ oder Wasserstoff und α-$CH_2OH$,

$X_2$ für $-CH_2-CH_2-$, $-C\equiv C-$ oder ein trans-Alkenylen

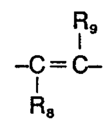

mit $R_8$ und $R_9$ in der oben angegebenen Bedeutung,

$X_3$ für 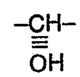 oder 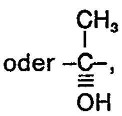,

$X_4$ für $-(CH_2)_n-$ mit n = 1–3 oder

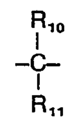

mit $R_{10}$ und $R_{11}$ als Wasserstoff, Fluor, Methyl, Methoxy oder mit $R_{10}$ und $R_{11}$ gemeinsam als $-CH_2-CH_2-$ oder $-CH_2-CH_2-CH_2-$,

$X_5$ für $-CH_2-$, Sauerstoff, Schwefel oder eine direkte Bindung und

$X_6$ für Phenyl, 3- oder 4-Chlorphenyl oder 3-Trifluormethylphenyl, oder für den Fall, dass $X_5$ eine direkte Bindung darstellt, für die Reste

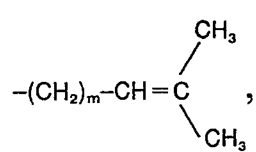,

$-(CH_2)_m-C\equiv C-CH_3$ oder $-(CH_2)_m-C\equiv C-CH_2-CH_3$ mit m = 1–2,

$-(CH_2)_4-OCH_3$, $-CH(CH_3)-CH_2-CH_2-CH_3$ oder $-(CH_2)_3-CH=CH_2$ steht, oder

$R_1$ einen Prostacyclinrest der Formel

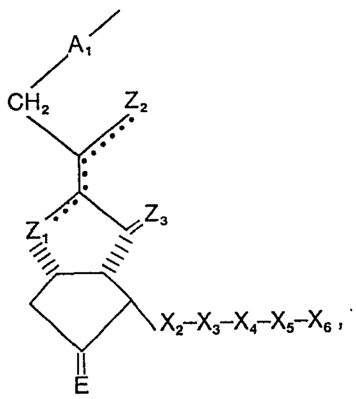

in dem $A_1$, E und $X_2$–$X_6$ die gleiche Bedeutung wie oben angegeben haben und

$Z_1$ Sauerstoff oder $CH_2$ darstellt, wenn gleichzeitig $Z_2$ Wasserstoff oder Cyano bedeutet, und $Z_1$ Stickstoff darstellt, wenn $Z_2$ die Bedeutung $H_2$ hat,

$Z_3H_2$ oder Sauerstoff oder

$R_1$ einen Prostacyclinrest der Formel

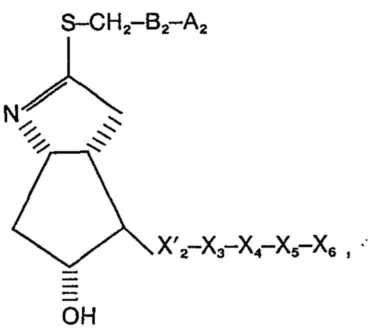

in dem $A_2$ die Gruppen

$$-CH_2-, -CF_2-, -\underset{CH_3}{\underset{|}{C}H}-$$

oder, wenn $B_2$ eine Einfachbindung darstellt, trans-Alkenylen

$$-\underset{R_8}{\underset{|}{C}}=\overset{R_9}{\overset{|}{C}}-,$$

$B_2$ $-CH_2$ oder eine Einfachbindung,

$X'_2$ $-CH_2-CH_2-$, $-C\equiv C-$ oder trans-Alkenylen

$$-\underset{R_8}{\underset{|}{C}}=\overset{R_9}{\overset{|}{C}}-$$

mit $R_8$ und $R_9$ als Wasserstoff oder Fluor, bedeuten und

$X_3$–$X_6$ die vorstehend angegebene Bedeutung haben, und

Y Sauerstoff, Schwefel oder Imino,

Q den Rest $(CR_6R_7)_p$, wobei p 0 bis 1 sein kann,

$R_2$ Wasserstoff, gegebenenfalls durch eine Hydroxy- oder Aminogruppe substituiertes Alkyl mit 1–6 C-Atomen, $C_1$–$C_4$-Alkoxycarbonyl, Cyano oder Di-$C_1$–$C_4$-alkylaminocarbonyl,

$R_3$, $R_4$, $R_5$, $R_6$, $R_7$ Wasserstoff oder Methyl,

$R_3$ und $R_4$ zusammen Trimethylen, Tetramethylen oder 1,3-Butadienylen, wenn $R_2$ und $R_5$ zusammen eine Direktbindung darstellen, bedeuten.

Für $R_2$ in der Bedeutung von Alkoxycarbonyl kommen folgende Alkylreste für Alk in Betracht: Methyl, Äthyl, Propyl, Isopropyl, Butyl, sek.-Butyl, tert.-Butyl und Benzyl.

In $-(CR_6R_7)_p-$ als Möglichkeit von Q kann p die Grösse 0 oder 1 haben.

Halogen in der Bedeutung von D kann Fluor, Chlor, Brom und Jod sein. Bevorzugt sind Fluor und Chlor.

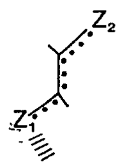

aus der allgemeinen Formel I hat bevorzugt folgende Bedeutungen:

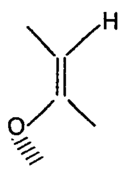 , 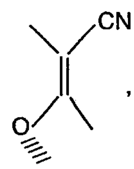 , 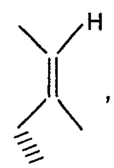 ,

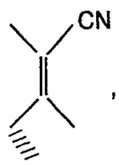 , 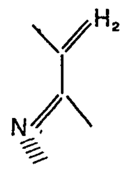 .

Die Erfindung betrifft ferner ein Verfahren zur Herstellung von Verbindungen der allgemeinen Formel I, dadurch gekennzeichnet, dass man eine Verbindung der allgemeinen Formel II,

$$R_1-COOR_{12} \qquad II,$$

worin $R_1$ die oben angegebene Bedeutung aufweist und $R_{12}$ Wasserstoff, Trialkylsilyl oder $C_1$–$C_6$-Alkyl bedeutet, gegebenenfalls nach Schutz anwesender freier Hydroxygruppen mit einem Amin der allgemeinen Formel III,

$$H-Y-Q-\underset{R_5}{\underset{|}{\overset{R_4}{\overset{|}{C}}}}-\underset{R_3}{\underset{|}{\overset{R_2}{\overset{|}{C}}}}-NH_2 \qquad III,$$

worin $R_2$, $R_3$, $R_4$, $R_5$, Q und Y die oben angegebenen Bedeutungen haben, mit Hilfe von organischen Phosphinen oder Phosphoniumsalzen und perhalogenierten Kohlenwasserstoffen oder Ketonen in Gegenwart von tertiären Basen umsetzt und gegebenenfalls anschliessend in beliebiger Reihenfolge Isomere trennt und/oder geschützte Hydroxygruppen freisetzt und/oder freie Hydroxygruppen verestert oder veräthert und/oder Hydroxygruppen oxidiert und/oder Oxogruppen reduziert und/oder Doppel- oder Dreifachverbindungen hydriert.

Die Verbindungen der allgemeinen Formel I, in denen $R_1$ einen Prostacyclinrest

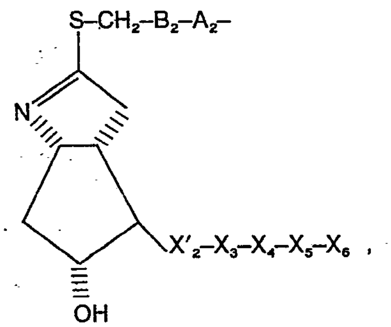

mit den für $A_2$, $B_2$, $X'_2$, $X_3$, $X_4$, $X_5$ und $X_6$ bereits angegebenen Bedeutungen darstellt, können auch ausgehend von den bicyclischen Thiolactonen

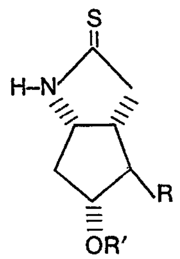

und

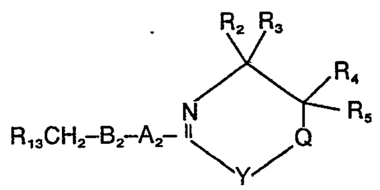

in $CH_2Cl_2$, Acetonitril oder N,N-Dimethylformamid, gegebenenfalls in Gegenwart einer tertiären Base, wie Triäthylamin bei 0–40 °C hergestellt werden. R und R′ stehen für die in der Prostacyclinchemie üblichen Reste, $R_{13}$ für eine austretende Gruppe wie z. B. Cl, Br, J, Mesyloxy oder Tosyloxy. $B_2$, $A_2$, Y, Q, $R_2$, $R_3$, $R_4$ und $R_5$ haben die bereits angegebene Bedeutung.

Als Äther- und Acylschutzgruppen für freie Hydroxyreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.butyl-silyl- und Tri-p-benzyl-silylrest. Als Acylreste kommen beispielsweise Acetyl, Propionyl, Butyryl, Benzoyl in Betracht.

Als Ausgangsprodukte II kommen alle Prostaglandine und Prostacycline, die sich auf $R_1$ zurückführen lassen und die in 1-Stellung eine $COOR_{12}$-Gruppe ($R_{12}$ z. B. H, $C_1$–$C_6$-Alkyl) aufweisen, in Betracht.

Bevorzugte Amine III für die Cyclisierung zu $\Delta^2$-N-Heterocyclen sind in der 5-Ring-Reihe
Äthanolamin, 2-Aminopropanol,
2-Methyl-2-amino-propanol,
Tris(hydroxymethyl)-methylamin,
o-Aminophenol, Cysteamin,
1,2-Äthylendiamin, o-Phenylendiamin,
1-Amino-2-methylaminoäthan,
1-Amino-2-phenylamino- oder -2-benzylaminoäthan,
und in der 6-Ring-Reihe
3-Aminopropanol,
2,3,3-Trimethyl-3-amino-1-propanol,
3-Aminopropanthiol, 1,3-Diaminopropan bzw.
1,3-Diamino-propane
der Formel

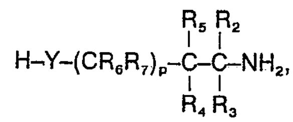

soweit sie noch nicht namentlich genannt worden sind. p bestimmt die Ringgrösse der $\Delta^2$-Heterocyclen der allgemeinen Formel I. So ergeben sich z. B.

für p = 0 $\Delta^2$-Oxazoline, $\Delta^2$-Thiazoline und $\Delta^2$-Imidazoline und

für p = 1 5,6-Dihydro-4H-1,3-oxazine, 5,6-Dihydro-4H-1,3-thiazine und Tetrahydro-pyrimidine.

Als organische Phosphine oder Phosphoniumsalze kommen in Betracht $[(R_{13})_3P^+{-}O{-}R_{14}]$-$ClO_4^-$, $CF_3SO_3^-$, $Cl^-Br^-$ oder $J^-$ und $(R_{13})_2\,P{-}R_{14}$ mit $R_{13}$ in der Bedeutung Aryl (Phenyl, α- oder β-Naphthyl, vorzugsweise Phenyl), Aralkyl (mit 7–10 C-Atomen wie bereits oben angegeben), Alkyl (mit 1–6 C-Atomen, siehe Rest für $R_2$), Cycloalkyl (mit 5–7 C-Atomen), O-Aryl (Phenyl, α- oder β-Naphthyl, vorzugsweise Phenyl, O-Alkyl (mit 1–6 C-Atomen, siehe Reste für $R_2$) und Di-($C_1$–$C_4$-alkyl)-amino (vorzugsweise Dimethylamino) bzw. als
$[(C_6H_5)_3P^+{-}O{-}P^+(C_6H_5)_3]2CF_3SO_3^-$ $[[(CH_3)_2N]_3P^+$-$O{-}P^+[N(CH_3)_2]_3]\ 2CF_3SO_3^-$ bzw.
$[[(CH_3)_2N]_3P^+{-}Cl]ClO_4^-$ d. h. $R_{14} = O{-}P^+(C_6H_5)_3$; $OP[N(CH_3)_2]$; Cl.

Da die Reaktionsgeschwindigkeit in der oben beschriebenen Reihenfolge abnimmt, ist $(R_{13})_3P$ für $P_{13}$ = Aryl, vorzugsweise Phenyl, an reaktivsten. Daher sind
$(C_6H_5)_3P/CCl_4$, $(C_6H_5)_3P/C_2Cl_6$,
$[(C_6H_5)_3P^+{-}O{-}P^+(C_6H_5)_3]2CF_3SO_3^-$ bzw.
$[(C_6H_5)_3P{-}Cl]Cl^-$;
$[(C_6H_5)_3P^+{-}Br]Br^-$; $[(C_6H_5)_3P^+{-}J]J^-$;
$(C_6H_5)_2P{-}Cl$ oder polymere aromatische Phosphine, in denen Triarylphosphine an eine polymere Matrix chemisch gebunden sind, bevorzugte Reagenzien.

Als elektrophile Komponente werden perhalogenierte Aliphaten und Aralkyle sowie Carbonylverbindungen angewandt wie
$CCl_4$, $CBrCl_3$, $CBr_2\,Cl_2$, $CClBr_3$, $CBr_4$, $C_2Cl_6$,
$C_6H_5{-}CCl_3$, $CCl_3{-}CO{-}CCl_3$, $CCl_3{-}CH_3$, $CHBr_3$,
$CCl_3\,CN$, $CCl_3{-}CHO$ usw. vorzugsweise aber $CCl_4$, als Azoester $R_{11}OOC{-}N{=}N{-}COOR_{11}$ mit $R_{11}$ $= CH_3, C_2H_5$,

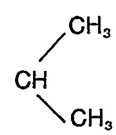

, $CH_2CCl_3$,

vorzugsweise mit $R_{11} = CH_3$, $C_2H_5$.

Als tert. Amine werden z. B.
Trimethylamin, Triäthylamin, Tri-n-propylamin,
Tributylamin, Diisopropyläthylamin,
Dicyclohexyläthylamin, Benzyldimethylamin,

Pyridin, Lutidin, Collidin,
2-Dimethylaminopyridin,
4-Dimethylaminopyridin, Chinolin,
1,4-Diazabicyclo [4,3,0]non-5-en (DBN),
1,8-Diazabicyclo[5,4,0]undec-7-en (DBU)
1,4-Diazabicyclo[2.2.2]octan (DABCO),
vorzugsweise Triäthylamin und Pyridin verwendet.

Die Reaktion wird in nicht-protischen absoluten Lösungsmitteln oder Lösungsmittelgemischen wie $CCl_4$, Chloroform, Methylenchlorid, Benzol, Toluol, Diäthyläther, Tetrahydrofuran, Essigester, Acetonitril, Dimethylformamid (DMF) oder Sulfolan, vorzugsweise in Acetonitril, Pyridin oder DMF durchgeführt.

Die Reaktion verläuft bei Temperaturen zwischen $-20\,°C$ und $100\,°C$, vorzugsweise bei $+10\,°C$ bis $+30\,°C$.

Es ist zweckmässig, pro Carboxyl- oder Estergruppe äquivalente Mengen der Aminkomponente (ω-Hydroxy- und ω-Mercaptoamin und ω-Diamin) zu verwenden. Das tert. Phosphin (vorzugsweise Triphenylphosphin) und das Elektrophil (vorzugsweise $CCl_4$ oder $C_2Cl_6$) werden in 2–5fachem molaren Überschuss, vorzugsweise 3–4fachem molaren Überschuss, bezogen auf die Carboxylgruppe eingesetzt. Von Phosphoniumsalzen wie $[(C_6H_5)_3P^+–O–P^+(C_6H_5)_3]\ 2CF_3SO_3^-$ benötigt man mindestens 2–3 Äquivalente.

Von dem tert. Amin (vorzugsweise Triäthylamin) verwendet man zweckmässigerweise ebenfalls 2–5 Äquivalente, vorzugsweise mindestens 4 Äquivalente. Ein Überschuss des Triäthylamins bewirkt nämlich eine bessere Löslichkeit der Aminsalze der Carbonsäuren.

Da die Reaktion von trisubstituierten Phosphinen $(R_{13})_3P$ (vorzugsweise Triphenylphosphin) mit Halogenverbindungen, vorzugsweise $CCl_4$, über eine ganze Serie von Reaktionsprodukten erfolgt [vgl. R. Appel, Angew. Chem. 87, 863 (1975)] und die ersten Reaktionsprodukte, z.B. $[(C_6H_5)_3P^+\text{-}CCl_3]Cl^-$ optimal für die Cyclisierung sind, ist es zweckmässig, das Triphenylphosphin in Lösung (vorzugsweise in Acetonitril) langsam zu dem Gemisch der anderen Reaktionspartner zuzutropfen, um hohe Ausbeuten der gewünschten Prostaglandine oder Prostacycline I zu erreichen.

Man kann diese Verbindungen an sehr desaktivierten Adsorbentien wie z.B. Aluminiumoxyd (A IV–V) oder Silicagel, das mit 30–40% Wasser versetzt wurde, möglichst unter Anwendung von Druck chromatographieren, ohne dass grössere Mengen dieser Substanzen bei der Chromatographie zersetzt werden.

Nach dem in der DE-OS 3 047 759 beschriebenen Verfahren lassen sich substituierte Carbonsäuren R′–COOH in Gegenwart von Aminen der allgemeinen Formel II sehr leicht mit tert. Phosphinen, insbesondere Triphenylphosphin, in Gegenwart von Halogenverbindungen wie insbesondere Tetrachlorkohlenstoff und einer tert. Base vorzugsweise Triäthylamin oder DBN, DBU bzw. mit $[(C_6H_5)_3P^+–O–P^+\ (C_6H_5)_3]\ 2CF_3SO_3^-$ zu den bereits erwähnten 2-substituierten $\Delta^2$-Oxazolinen, $\Delta^2$-Imidazolinen bzw. 5,6-Dihydro-4H-1,3-oxazinen, 5,6-Dihydro-4H-thiazinen oder Tetrahydropyrimidinen überführen. Dabei bilden sich stets die noch nicht cyclisierten ω-Hydroxy-, ω-Mercapto- oder ω-Aminoalkylamide als isolierbare Zwischenprodukte, die wie die Carbonsäuren zur Cyclisation eingesetzt werden können.

So lässt sich z.B. Prostaglandin $-F_2\alpha$ sehr leicht nach Schutz der reaktiven Hydroxylgruppen durch Silylierung (falls diese Hydroxylgruppen nicht sowieso schon durch andere Schutzgruppen wie Acyl, Tetrahydropyranyl oder Silylgruppen blokkiert sind) direkt mit Äthanolamin in Gegenwart von Triphenylphosphin, Tetrachlorkohlenstoff und Triäthylamin in absolutem Acetonitril oder N,N-Dimethylformamid zum persilylierten $\Delta^2$-Oxazolin umsetzen, aus dem sich durch wässrige oder alkoholische Lauge die Trimethylsilylschutzgruppen in 9-, 11- und 15-Stellung leicht entfernen lassen.

Man kann aber auch die Ausgangs-Prostaglandine und -Prostacycline der Formel II sowie ihre Analoga direkt ohne Schutz ihrer Hydroxygruppen unter sorgfältig kontrollierten Bedingungen mit Triphenylphosphin, Tetrachlorkohlenstoff und Triäthylamin insbesondere in abs. Acetonnitril, Acetonnitril-Pyridin, N,N-Dimethylformamid oder N-Methylpyrolidon oder Sulfolan zu den entsprechenden Derivaten der 1-Carboxylgruppe wie $\Delta^2$-Oxazoline, $\Delta^2$-Thiazoline, $\Delta^2$-Imidazoline sowie $\Delta^2$-Oxazine usw. umsetzen, ohne dass diese freien Hydroxylgruppen der Ausgangssubstanzen verändert bzw. in ihre entsprechende Chlorderivate umgewandelt werden.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuss angewandt, vorzugsweise in der 4- bis 10fachen Menge des theoretischen Bedarfs. Die Umsetzung ist normalerweise bei 0–30 °C nach 15–30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wässrigen Lösung einer organischen Säure, wie zum Beispiel Essigsäure, Propionsäure u.a. oder in einer wässrigen Lösung einer anorganischen Säure, wie zum Beispiel Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10 °C bis 70 °C, vorzugsweise bei 25 °C.

Die Oxydation anwesender Hydroxygruppen wird nach an sich bekannten Methoden mit den üblichen Oxydationsmitteln vorgenommen. Beispielsweise kann die Oxydation der 9-Hydroxygruppe zum 9-Keton mit Jones-Reagenz (J. Chem. Soc. 1953, 2555) erfolgen, wobei weitere freie Hydroxylgruppen im Molekül, z.B. in 11- und/oder 15-Stellung, vorher in bekannter Weise selektiv geschützt werden. Man arbeitet mit einem Überschuss des Oxydationsmittels in einem inerten Lösungsmittel, wie Aceton, bei Temperaturen zwischen 30 °C und −50 °C, vorzugsweise bei etwa −20 °C. Die Reaktion ist allgemein nach etwa 5 bis 30 Minuten beendet.

Die Reduktion der 9-Ketogruppe zur Herstellung der entsprechenden 9β-Hydroxyverbindungen erfolgt mit einem für die Reduktion von Ketonen geeigneten Reduktionsmittel, wie beispielsweise Natriumborhydrid. Das entstehende Epimerengemisch wird zum Beispiel in üblicher Weise durch Säulen- oder Schichtchromatographie getrennt.

Sollen im Primärprodukt enthaltene C=C-Doppelbindungen reduziert werden, erfolgt die Hydrierung nach an sich bekannten Methoden.

Die Hydrierung der 5,6-Doppelbindung wird in an sich bekannter Weise bei tiefen Temperaturen, vorzugsweise bei etwa −20 °C, in einer Wasserstoffatmosphäre in Gegenwart eines Edelmetallkatalysators durchgeführt. Als Katalysator ist zum Beispiel 10% Palladium auf Kohle geeignet.

Wird sowohl die 5,6- als auch die 13,14-Doppelbindung hydriert, so arbeitet man bei höherer Temperatur vorzugsweise bei etwa 20 °C.

Sofern die als Ausgangsmaterial dienenden Ester der allgemeinen Formel II nicht bekannt sind, lassen sie sich leicht aus den bekannten Carbonsäuren durch Umsetzung mit Diazoalkanen in einem inerten Lösungsmittel, vorzugsweise in Diäthyläther oder Methylenchlorid herstellen.

Die Trialkylsilylester ($R_{12}$ = Trialkylsilyl) entstehen in der Regel bei der Trialkylsilyläther-Bildung der zu schützenden OH-Gruppen.

Die neuen Prostaglandin-Analoga der Formel I wirken sehr stark luteolytisch, d.h. zur Auslösung einer Luteolyse benötigt man wesentlich geringere Dosierungen als bei den entsprechenden natürlichen Prostaglandinen.

Auch zur Auslösung von Aborten, insbesondere nach oraler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine.

Die erfindungsgemässen Wirkstoffe der PGE-Reihe zeigen an der isolierten Kaninchen-Trachea in vitro sogar eine bronchodilatorische Wirkung und hemmen stark die Magensäuresekretion.

Die Prostacycline dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch grössere Stabilität aus. Die hohe Gewebsspezifität der neuen Prostaglandine zeigt sich bei der Untersuchung an glattmuskulären Organen, wie z.B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostacyclin-Analoga besitzen die für Prostacycline typischen Eigenschaften, wie z.B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes

ohne zugleich Schlagvolumen und koronare Durchblutung zu senken und sind zur Behandlung von Schlaganfall geeignet.

Beispiel 1
(5Z,13E)-(8R,9S,11R,12R,15S)-9,11,15-Triacetoxy-2-(2-oxazolin-2-yl)-1-nor-5,13-prostadien

207 mg (0,43 mMol) 9,11,15-Triacetyl-prostaglandin-$F_{2\alpha}$ werden in 2 ml abs. Acetonitril gelöst und mit 0,43 ml einer molaren Lösung von Äthanolamin in Acetonitril, 394 mg (1,5 mMol) Triphenylphosphin, und 5,3 ml einer molaren Lösung von Triäthylamin in Acetonitril versetzt. Die schwach gelb gefärbte klare Lösung wird auf 2 °C Innentemperatur gekühlt. Innerhalb von 5 Minuten werden 0,86 ml einer molaren Lösung von Tetrachlorkohlenstoff in Acetonitril zugetropft und anschliessend 4 Stunden bei 2 °C gerührt. Nach 48 Stunden stehen bei Raumtemperatur wird das Reaktionsgemisch ohne Erwärmung eingeengt und viermal mit je 15 ml dest. Hexan extrahiert. Nach Einengen und Kristallisieren des Triphenylphosphinoxids aus Hexan erhält man 196 mg (90,2% der Theorie) der Titelverbindung.

Beispiel 2
(5Z,13E)-(8R,9S,11R,12R,15S)-2-(2-Oxazolin-2-yl)-1-nor-5,13-prostadien-9,11,15-triol

516 mg (1mMol) der nach Beispiel 1 hergestellten Verbindung werden in 15 ml Methanol gelöst, die Lösung auf 0 °C abgekühlt und mit 15 ml 2nNaOH versetzt. Nach 30 Minuten Rühren im Eisbad wird 2 Stunden bei Raumtemperatur gerührt. Nach schonendem Einengen der Reaktionslösung am Rotationsdampfer bei 25 °C auf ca. 10 ml wird mit 10 ml Wasser verdünnt und viermal mit je 20 ml Äthylacetat extrahiert. Nach Trocknen über $Na_2SO_4$ und Einengen wird die im Methylenchlorid aufgenommene Substanz präparativ auf Silikagel mit Chloroform/Methanol (9:1) als Laufmittel getrennt, zunächst mit Äthanol und dann dreimal mit wenig Tetrachlorkohlenstoff eluiert. Ausbeute 178 mg (46,9% der Theorie).

Beispiel 3
(5Z,13E)-(8R,9S,11R,12R,15S)-2-(2-Oxazolin-2-yl)-1-nor-5,13-prostadien-9,11,15-triol

177 mg (0,5 mMol) Prostaglandin-$F_{2\alpha}$ werden in 5 ml dest. Hexamethyldisilazan (HMDS) suspendiert, wobei sich unter $NH_3$-Entwicklung und Erwärmung nach 30 Minuten eine klare farblose Lösung bildet. Nach 1,5 Stunden Erhitzen bei 140° Badtemperatur wird überschüssiges HMDS im Vakuum abgezogen, und der Rückstand 30 Minuten bei 40° Badtemperatur und 0,2 mBar an der Ölpumpe getrocknet.

Der Rückstand wird in 5 ml abs. Acetonitril gelöst, 786 mg (3mMol) Triphenylphosphin und 1,05 ml (7,5mMol) Triäthylamin zugegeben und unter Eiskühlung 0,5 ml einer molaren Lösung von Äthanolamin in Acetonitril zugetropft. Danach erfolgt Zugabe von 1,5 ml einer molaren Lösung von Tetrachlorkohlenstoff in Acetonitril und Stehen über Nacht bei Raumtemperatur. Nach Einengen im Vakuum wird fünfmal mit je 75 ml Hexan gewaschen. Die gebildeten Kristalle werden vom öligen Rückstand abgetrennt, und der ölige Rückstand in 15 ml Methanol aufgenommen und unter Kühlung mit 5 ml 2n NaOH versetzt, 30 Minuten bei 20° gerührt. Nach Einengen im Vakuum auf ca. 5 ml wird mit 10 ml Wasser versetzt und viermal mit je 10 ml Äthylacetat extrahiert. Nach Trocknen der Äthylacetat-Phasen mit $Na_2SO_4$ und Einengen bleiben 208 mg eines hellbraunen öligen Rückstandes zurück. Nach präparativer Dünnschichtchromatographie an $SiO_2$ mit Chloroform/Methanol (9:1) als Laufmittel wird mit 300 ml Äthanol eluiert, eingeengt und an der Ölpumpe 1 Stunde bei 20° und 1,5 mBar getrocknet. Ausbeute 103 mg (54,2% der Theorie).

Beispiel 4
(5Z,13E)-(8R,9S,11R,12R,15S)-2-(2-Oxazolin-2-yl)-1-nor-5,13-prostadien-9,11,15-triol

177 mg (0,5 mMol) Prostaglandin-$F_{2\alpha}$ werden in 15 ml abs. Acetonitril gelöst und unter Argon mit 0,7 ml Triäthylamin, 0,5 ml einer molaren Lösung von Äthanolamin in Acetonitril und 0,5 ml Tetrachlorkohlenstoff versetzt. Innerhalb von 8 Stunden werden bei ca. 20 °C 655 mg (2,5 mMol) Triphenylphosphin in 15 ml abs. Acetonitril unter Rühren zugetropft. Anschliessend wird noch 20 Stunden bei 20 °C gerührt. Nach Einengen im Vakuum wird der Rückstand in 20 ml Äthylacetat und 15 ml Wasser aufgenommen und die wässrige Phase dreimal mit je 10 ml Äthylacetat nachextrahiert. Die vereinigten Essigester-Phasen werden über $Na_2SO_4$ getrocknet und eingeengt. Die Chromatographie der Essigesterlösung (10 ml) mit wassergesättigtem Essigester an $Al_2O_3$ (basisch, Akt. IV) ergibt 127 mg (66,9%) der Titelverbindung als Öl.

Beispiel 5
(5Z,13E)-(8R,9S,11R,12R,15S)-2-(4,4-Dimethyl-2-oxazolin-2-yl)-1-nor-5,13-prostadien-9,11,15-triol

Analog zu Beispiel 3 werden 177 mg (0,5 mMol) Prostaglandin-$F_{2\alpha}$ mit 0,5 ml einer molaren Lösung von 2-Amino-2-methyl-1-propanol in Acetonitril umgesetzt. Ausbeute 98 mg (48,3% der Theorie).

Beispiel 6
(5Z,13E)-(8R,9S,11R,12R,15S)-2-(2-Thiazolin-2-yl)-1-nor,5,13-prostadien-9,11,15-triol

Analog Beispiel 3 aus 177 mg (0,5 mMol) Prostaglandin-$F_{2\alpha}$ und 57 mg (0,5 mMol) 2-Aminoäthanthiol-hydrochlorid unter Argon. Ausbeute 113 mg (57% der Theorie).

Beispiel 7
1-Decarboxy-2-(oxazolin-2-yl)-(5R,6R)-5-brom-prostaglandin-$I_1$

Analog Beispiel 4 aus 216 mg (0,5 mMol) (5R,6R)-5-Brom-prostaglandin-$I_1$ und Äthanolamin. Ausbeute 125 mg (54,4% der Theorie).

Beispiel 8
1-Decarboxy-2-(oxazolin-2-yl)prostaglandin-$I_2$

125 mg (0,27 mMol) der nach Beispiel 7 hergestellten Verbindung werden mit 5 ml abs. Toluol versetzt und unter Argon 0,25 ml DBU zugegeben. Nach 8 Stunden Rühren bei 60–65 °C, Einengen, Chromatographie an $SiO_2$ mit Essigester/Methanol (9:1) entstehen 43 mg (42,3% der Theorie) eines hellgelben zähen Öls.

Beispiel 9

2-[4-(E)-(1S,5S,6R,7R)-7-Hydroxy-6-[(E)-(3S,4RS)-3-hydroxy-4-methyl-oct-1-en-6-in-yl]-bicyclo[3.3.0]-octan-3-yliden-butyl]-2-oxazolin

Analog Beispiel 4 aus 50 mg (0,14 mMol) 5-{(E)-(1S,5S,6R,7R)-7-Hydroxy-6-[(E)-(4RS)-3α-hydroxy-4-methyl-oct-1-en-6-inyl]-bicyclo[3.3.0]octan-3-yliden}-pentansäure und Äthanolamin unter Argon. Ausbeute 16 mg (29,6% der Theorie).

Beispiel 10

2-{(E)-(1S,5R,6R)-7-Hydroxy-6-[(E)-3S,4RS)-3-hydroxy-4-methyl-1-octenyl]-2-oxabicyclo-[3.3.0]octan-3-yliden}-5-(2-oxazolin-2-yl)-pentannitril

Analog Beispiel 4 aus 78 mg (0,2 mMol) 5-Cyan-5-{(1S,5R,6R)-7-hydroxy-6-[(E)-(3S,4RS)-3-hydroxy-4-methyl-1-octenyl]-2-oxabicyclo[3.3.0]octan-3-yliden}-pentansäure und Äthanolamin. Ausbeute 60 mg (72% der Theorie).

Beispiel 11

2-Aza-3-[1-thia-4-(2-oxazolin-2-yl)-butyl]-6-(3α-acetoxy-1-octenyl)-7α-acetoxy-bicyclo[3.3.0]-octen-2

195 mg (0,43 mMol) 2-Aza-3-(1-thia-4-carboxybutyl)-6-(3α-acetoxy-1-octenyl)-7α-acetoxy-bicyclo[3.3.0]octen-2 werden in 2 ml abs. Acetonitril gelöst und mit 0,43 ml einer molaren Lösung von Äthanolamin in Acetonitril, 394 mg (1,5 mMol) Triphenylphosphin, und 5,3 ml einer molaren Lösung von Triäthylamin in Acetonitril versetzt. Die schwach gelb gefärbte klare Lösung wird auf 2 °C Innentemperatur gekühlt. Innerhalb von 5 Minuten werden 0,86 ml einer molaren Lösung von Tetrachlorkohlenstoff in Acetonitril zugetropft und anschliessend 4 Stunden bei 2 °C gerührt. Nach 48 Stunden Stehen bei Raumtemperatur wird das Reaktionsgemisch ohne Erwärmung eingeengt und viermal mit je 15 ml dest. Hexan extrahiert. Nach Einengen und Kristallisieren des Triphenylphosphinoxids aus Hexan erhält man 172,8 mg (80,6% der Theorie) der Titelverbindung.

Beispiel 12

2-Aza-3-[1-thia-4-(2-oxazolin-2-yl)-butyl]-6-(3α-hydroxy-1-octenyl)-7α-hydroxy-bicyclo[3.3.0]octen-2

498,6 mg (1 mMol) der nach Beispiel 1 hergestellten Verbindung werden in 15 ml Methanol gelöst, die Lösung auf 0 °C abgekühlt und mit 15 ml 2nNaOH versetzt. Nach 30 Minuten Rühren im Eisbad wird 2 Stunden bei Raumtemperatur gerührt. Nach schonendem Einengen der Reaktionslösung am Rotationsdampfer bei 25 °C auf ca. 10 ml wird mit 10 ml Wasser verdünnt und viermal mit je 20 ml Äthylacetat extrahiert. Nach Trocknen über $Na_2SO_4$ und Einengen wird die im Methylenchlorid aufgenommene Substanz präparativ auf Silikagel mit Chloroform/Methanol (9:1) als Laufmittel getrennt, zunächst mit Äthanol und dann dreimal mit wenig Tetrachlorkohlenstoff eluiert. Ausbeute 213,5 mg (54,1% der Theorie).

Beispiel 13

2-Aza-3-[1-thia-4-(2-oxazolin-2-yl)-butyl]-6-(3α-trimethylsilyloxy-1-octenyl)-7α-trimethylsilyloxy-bicyclo-[3.3.0]octen-2

184,7 mg (0,5 mMol) 2-Aza-3-(1-thia-4-carboxybutyl)-6-(3α-hydroxy-1-octenyl)-7α-hydroxy-bicyclo[3.3.0]octen-2 werden in 5 ml dest. Hexamethyldisilazan (HMDS) suspendiert, wobei sich unter $NH_3$-Entwicklung und Erwärmung nach 30 Minuten eine klare farblose Lösung bildet. Nach 1,5 Stunden Erhitzen bei 140 °C Badtemperatur wird überschüssiges HMDS im Vakuum abgezogen, und der Rückstand 30 Minuten bei 40 °C Badtemperatur und 0,2 mBar an der Ölpumpe getrocknet.

Der Rückstand wird in 5 ml abs. Acetonitril gelöst, 786 mg (3 mMol) Triphenylphosphin und 1,05 ml (7,5 mMol) Triäthylamin zugegeben und unter Eiskühlung 0,5 ml einer molaren Lösung von Äthanolamin in Acetonitril zugetropft. Danach erfolgt Zugabe von 1,5 ml einer molaren Lösung von Tetrachlorkohlenstoff in Acetonitril und Stehen über Nacht bei Raumtemperatur. Nach Einengen im Vakuum wird fünfmal mit je 75 ml Hexan gewaschen. Die gebildeten Kristalle werden abgetrennt und der ölige Rückstand 1 Stunde bei 20 °C und 1,5 mBar getrocknet. Ausbeute 234,7 mg (87,1% der Theorie).

Beispiel 14

2-Aza-3-[1-thia-4-(2-oxazolin-2-yl)-butyl]-6-(3α-hydroxy-1-octenyl)-7α-hydroxy-bicyclo[3.3.0]octen-2

215,6 mg (0,4 mMol der nach Beispiel 3 hergestellten Verbindung werden in 15 ml Methanol aufgenommen und unter Kühlung mit 5 ml 2n NaOH versetzt und danach 30 Minuten bei 20 °C gerührt. Nach Einengen im Vakuum auf ca. 5 ml wird mit 10 ml Wasser versetzt und viermal mit je 10 ml Äthylacetat extrahiert. Nach Trocknen der Äthylacetat-Phasen mit $Na_2SO_4$ und Einengen bleiben 208 mg eines hellbraunen öligen Rückstandes zurück. Nach präparativer Dünnschichtchromatographie an $SiO_2$ mit Chloroform/Methanol (9:1) als Laufmittel wird mit 300 ml Äthanol eluiert, eingeengt und an der Ölpumpe 1 Stunde bei 20 °C und 1,5 mBar getrocknet. Ausbeute 90,3 mg (57,2% der Theorie).

Beispiel 15

2-Aza-3-[1-thia-4-(2-oxazolin-2-yl)-butyl]-6-(3α-hydroxy-4(R,S)-methyl-1-

octenyl)-7α-hydroxy-bicyclo[3.3.0]-octen 2

Analog Beispiel 1 aus 187 mg (0,4 mMol) 2-Aza-3-(1-thia-4-carboxybutyl)-6-(3α-acetoxy-4(R,S)-methyl-1-octenyl)-7α-acetoxy-bicyclo[3.3.0]octen-2 und anschliessender Verseifung des isolierten 2-Aza-3-[1-thia-4-(2-oxazolin-2-yl)-butyl]-6-(3α-acetoxy-4(R,S)-methyl-1-octenyl)-7α-acetoxy-bicyclo[3.3.0]-octens-2 mit NaOH/$CH_3OH$ analog Beispiel 2. Ausbeute 80,9 mg (49,5% der Theorie).

Beispiel 16

2-Aza-3-[1-thia-4-(2-oxazolin-2-yl)-butyl]-6-(3α-hydroxy-4,4-dimethyl-1-octenyl)-7α-hydroxy-bicyclo[3.3.0]-octen-2

Analog Beispiel 1 aus 192,65 mg (0,4 mMol) 2-Aza-3-(1-thia-4-carboxybutyl)-6-(3α-acetoxy-4,4-dimethyl-1-octenyl)-7α-acetoxy-bicyclo[3.3.0]octen-2 und anschliessende Verseifung der Bis-acetoxyverbindung analog Beispiel 1. Ausbeute 70,8 mg (41,9% der Theorie).

Beispiel 17

2-Aza-3-[1-thia-4-(2-oxazolin-2-yl)-butyl]-6-(3α-hydroxy-4-methyl-6,7-tetrahydro-1-nonenyl)-7α-hydroxy-bicyclo[3.3.0]octen-2

Analog Beispiel 1 aus 143,3 mg (0,3 mMol) 2-Aza-3-(1-thia-4-carboxybutyl)-6-(3α-acetoxy-4-methyl-6,7-tetradehydro-1-nonenyl)-7α-acetoxy-bicyclo[3.3.0]-octen-2 und anschliessende Verseifung der Bis-acetoxyverbindung analog Beispiel 2. Ausbeute 60 mg (47,8% der Theorie).

Beispiel 18

2-Aza-3-{1-thia-4-[2-(5,6-dihydro-4-H-1,3-oxazin-2-yl)]-butyl}-6-(3α-hydroxy-4-phenoxy-1-butenyl)-7α-hydroxy-bicyclo[3.3.0]octen-2

Analog Beispiel 1 aus 244,8 mg (0,5 mMol) 2-Aza-3-(1-thia-4-carboxybutyl)-6-(3α-acetoxy-4-phenoxy-1-butenyl)-7α-acetoxy-bicyclo[3.3.0]octen-2 und 3-Aminopropanol und anschliessende Verseifung der Bis-acetoxyverbindung, analog Beispiel 2. Ausbeute 120,7 mg (54,3% der Theorie).

Beispiel 19

2-Aza-3-[1-thia-3,3-difluor-4-(2-thiazolin-2-yl)-butyl]-6-(3α-hydroxy-5-phenyl-1-pentenyl)-7α-hydroxy-bicyclo-[3.3.0]-octen-2

Analog Beispiel 1 aus 261,8 mg (0,5 mMol) 2-Aza-3-(1-thia-3,3-difluor-4-carboxy-butyl)-6-(3α-acetoxy-4-phenyl-1-pentenyl)-7α-acetoxy-bicyclo[3.3.0]-octen-2 und Cysteamin und anschliessende Verseifung der Bis-acetoxyverbindung analog Beispiel 2. Ausbeute 126,2 mg (51,9% der Theorie).

Beispiel 20:

2-Aza-3-[1-thia-4-(2-imidazolin-2-yl)-butyl]-6-[3α-hydroxy-4-(3-chlorphenoxy)-1-butinyl]-7α-hydroxy-bicyclo[3.3.0]-octen-2

Analog Beispiel 1 aus 261 mg (0,5 mMol) 2-Aza-3-(1-thia-4-carboxybutyl)-6-[3α-acetoxy-4-(3-chlorphenoxy)-1-butinyl]-7α-acetoxy-bicyclo[3.3.0]octen-2 und 1,2-Diaminoäthan und anschliessende Verseifung der Bis-acetoxyverbindung analog Beispiel 2. Ausbeute 112,7 mg (48,8% der Theorie).

**Patentansprüche**

1. Prostaglandine der allgemeinen Formel I

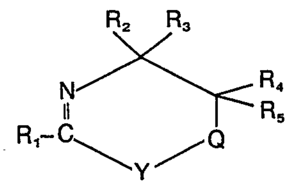

I,

in der

$R_1$ einen Prostaglandinrest der Formel

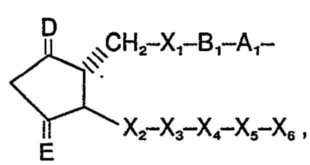

wobei

$A_1$ für $-(CH_2)_n-$, $-CF_2-(CH_2)_m-$ mit $n = 1-3$ und $m = 1-2$ und $-CH=CH-CH_2-$, $-CH_2-CH=CH-$ und

$B_1-B_1$ für $-(CH_2)_3-$,

$A_1-B_1-X_1$ für $-(CH_2)_5-$,

$A_1-B_1$ für $-(CH_2)_o-$, $-CF_2-(CH_2)_n$, $-CH=CH-(CH_2)_m-$, $-(CH_2)_m-CH=H-$ mit $m = 1-2$, $n = 1-3$ und $o = 1-4$ und

$X_1$ für ein cis-Alkenylen

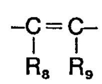

mit $R_8$ und $R_9$ als Wasserstoff odor $C_1-C_6$-Alkyl sowie

$A_1$ für $-CH_2-$,

$B_1$ für Sauerstoff oder $-CH_2-$,

D für Sauerstoff, Wasserstoff und α- oder β-Hydroxy, Wasserstoff und α- oder β-Halogen oder $CH_2$,

E für Sauerstoff, Wasserstoff und α-Hydroxy, Wasserstoff und α-$CH_3$ oder Wasserstoff und α-$CH_2OH$,

$X_2$ für $-CH_2-CH_2-$, $-C\equiv C-$ oder ein trans-Alkenylen

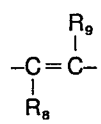

mit $R_8$ und $R_9$ in der oben angegebenen Bedeutung,

$X_3$ für

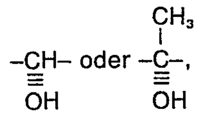

$X_4$ für $-(CH_2)_n-$ mit n = 1–3 oder

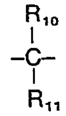

mit $R_{10}$ und $R_{11}$ als Wasserstoff, Fluor, Methyl, Methoxy oder mit $R_{10}$ und $R_{11}$ gemeinsam als $-CH_2-CH_2-$ oder $-CH_2-CH_2-CH_2-$,

$X_5$ für $-CE_2-$, Sauerstoff, Schwefel oder eine direkte Bindung und

$X_6$ für Phenyl, 3- oder 4-Chlorphenyl oder 3-Trifluormethylphenyl, oder für den Fall, dass $X_5$ eine direkte Bindung darstellt, für die Reste

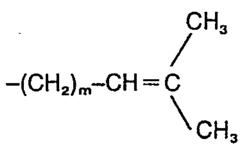 ,

$-(CH_2)_m-C\equiv C-CH_3$ oder $-(CH_2)_m-C\equiv C-CH_2-CH_3$ mit m = 1–2,

$-(CH_2)_4-OCH_3$, $-CH(CH_3)-CH_2-CH_2-CH_3$ oder $-(CH_2)_3-CH=CH_2$ steht, oder

$R_1$ einen Prostacyclinrest der Formel

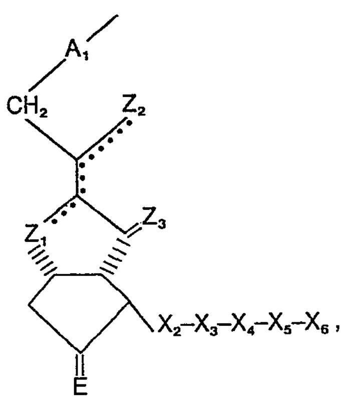

in dem $A_1$, E und $X_2$–$X_6$ die gleiche Bedeutung wie oben angegeben haben und

$Z_1$ Sauerstoff oder $CH_2$ darstellt, wenn gleichzeitig $Z_2$ Wasserstoff oder Cyano bedeutet, und $Z_1$ Stickstoff darstellt, wenn $Z_2$ die Bedeutung $H_2$ hat,

$Z_3H_2$ oder Sauerstoff oder

$R_1$ einen Prostacyclinrest der Formel

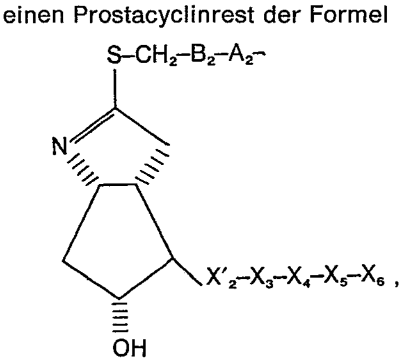

in dem $A_2$ die Gruppen 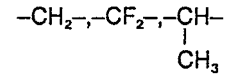

oder, wenn $B_2$ eine Einfachbindung darstellt, trans-Alkenylen

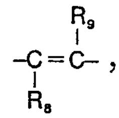 ,

$B_2$ $-CH_2$ oder eine Einfachbindung,

$X'_2$ $-CH_2-CH_2-$, $-C\equiv C-$ oder trans-Alkenylen

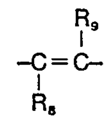

mit $R_8$ und $R_9$ als Wasserstoff oder Fluor bedeuten und $X_3$–$X_6$ die vorstehend angegebene Bedeutung haben, und

Y Sauerstoff, Schwefel oder Imino,

Q den Rest $(CR_6R_7)_p$, wobei p 0 bis 1 sein kann,

$R_2$ Wasserstoff, gegebenenfalls durch eine Hydroxy- oder Aminogruppe substituiertes Alkyl mit 1–6 C-Atomen, $C_1$–$C_4$-Alkoxycarbonyl, Cyano oder Di-$C_1$1$C_4$-alkylaminocarbonyl,

$R_3$, $R_4$, $R_5$, $R_6$, $R_7$ Wasserstoff oder Methyl,

$R_3$ und $R_4$ zusammen Trimethylen, Tetramethylen oder 1,3-Butadienylen, wenn $R_2$ und $R_5$ zusammen eine Direktbindung darstellen, bedeuten.

2. (5Z,13E)-(8R,9S,11R,12R,15S)-2-(2-Oxazolin-2-yl)-1-nor-5,13-prostadien-9,11,15-triol.

3. 2-[4-(E)-(1S,5S,6R,7R)-7-Hydroxy-6-[(E)-(3S,4RS)-3-hydroxy-4-methyl-oct-1-en-6-in-yl]-bicyclo[3.3.0]-octan-3-yliden)-butyl]-2-oxazolin.

4. 2-{(E)-(1S,5R,6R)-7-Hydroxy-6-[(E)-(3S,4RS)-3-hydroxy-4-methyl-1-octenyl]-2-oxabicyclo[3.3.0]octan-3-yliden}-5-(2-oxazolin-2-yl)-pentannitril.

5. Verfahren zur Herstellung von Verbindungen der allgemeinen Formel I, dadurch gekennzeichnet, dass man Verbindungen der allgemeinen Formel II

$$R_1-COOR_{12} \qquad (II),$$

worin $R_1$ die oben angegebene Bedeutung aufweist und $R_{12}$ Wasserstoff, Trialkylsilyl oder $C_1$–$C_6$-Alkyl bedeutet, gegebenenfalls nach Schutz anwesender freier Hydroxygruppen mit einem Amin der allgemeinen Formel III,

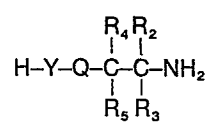 III,

worin $R_2$, $R_3$, $R_4$, $R_5$, Q und Y die oben angegebenen Bedeutungen haben, mit Hilfe von organischen Phosphinen oder Phosphoniumsalzen und perhalogenierten Kohlenwasserstoffen oder Ketonen in Gegenwart von tertiären Basen umsetzt und gegebenenfalls anschliessend Isomere trennt und/oder geschützte Hydroxygruppen freisetzt und/oder freie Hydroxygruppen verestert und veräthert und/oder Hydroxygruppen oxidiert und/

oder Oxogruppen reduziert und/oder Doppel- oder Dreifachbindungen hydriert.

Arzneimittel, bestehend aus einer oder mehreren Verbindungen gemäss Anspruch 1 und üblichen Hilfs- und Trägerstoffen.

**Claims**

1. Prostaglandins of the general formula I

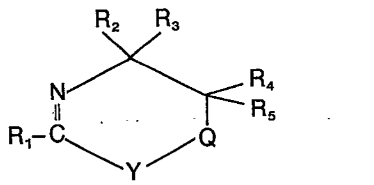
I,

in which

$R_1$ is a prostaglandin residue of the formula

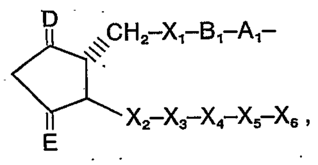

in which

$A_1$ is $-(CH_2)_n-$, $-CF_2-(CH_2)_m-$, where n = 1–3 and m = 1–2, $-CH=CH-CH_2-$ or $-CH_2-CH=CH-$, and

$B_1-X_1$ is $-(CH_2)_3-$,

$A_1-B_1-X_1$ is $-(CH_2)_5-$,

$A_1-B_1$ is $-(CH_2)_o-$, $-CF_2-(CH_2)_n-$, $-CH=CH-(CH_2)_m$, $-(CH_2)_m-CH=CH-$, where m = 1–2, n = 1–3 and o = 1–4, or

$X_1$ is a cis alkenylene,

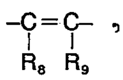

where $R_8$ and $R_9$ are hydrogen or $C_1$–$C_6$-alkyl as well as

$A_1$ is $-CH_2-$, and

$B_1$ is oxygen or $-CH_2$,

D is oxygen, hydrogen and α- or β-hydroxy, hydrogen and α- or β-halogen or $CH_2$,

E is oxygen, hydrogen and α-hydroxy, hydrogen and α-$CH_3$ or hydrogen and α-$CH_2OH$,

$X_2$ is $-CH_2-CH_2-$, $-C\equiv C-$ or a trans-alkenylene

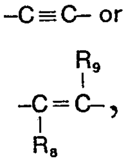

where $R_8$ and $R_9$ have the meanings given above,

$X_3$ is

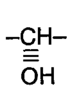 oder 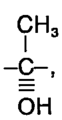

$X_4$ is $-(CH_2)_n$, where n = 1–3, or

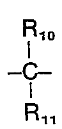

where $R_{10}$ and $R_{11}$ are hydrogen, fluoro, methyl, methoxy or $R_{10}$ and $R_{11}$ are together $-CH_2-CH_2-$ or $-CH_2-CH_2-CH_2$,

$X_5$ is $-CH_2-$, oxygen, sulphur or a direct bond and

$X_6$ is phenyl, 3- or 4-chlorophenyl or 3-trifluoromethylphenyl, or in the case when $X_5$ is a direct bond is

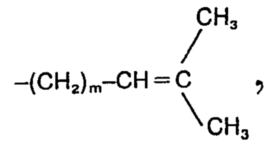

$-(CH_2)_m-C\equiv C-CH_3$ or $-(CH_2)_m-C\equiv C-CH_2-CH_3$, where m = 1–2,

$-(CH_2)_4-OCH_3$, $-CH(CH_3)-CH_2-CH_2-CH_3$ or $-(CH_2)_3-CH=CH_2$, or

$R_1$ is a prostacyclin residue of formula

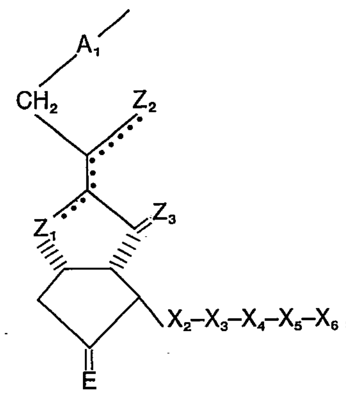

in which $A_1$, E and $X_2$–$X_6$ have the same meanings given above and

$Z_1$ is oxygen or $CH_2$, when $Z_2$ is hydrogen or cyano, and $Z_1$ is nitrogen when $Z_2$ is $H_2$,

$Z_3$ is $H_2$ or oxygen, or

$R_1$ is a prostaglandin residue of formula

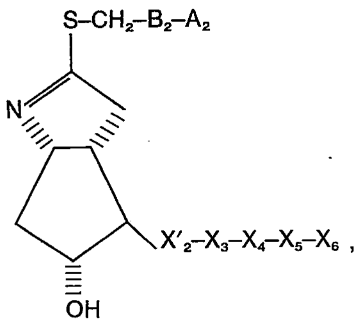

in which $A_2$ is $-CH_2-$, $-CF_2-$, 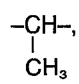, or is trans alkenylene

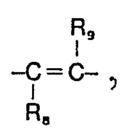

when $B_2$ is a single bond,

$B_2$ is $-CH_2-$ or a single bond,

$X_2$ is $-CH_2-CH_2-$, $-C\equiv C-$, or trans-alkenylene

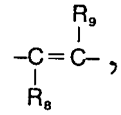

where $R_8$ and $R_9$ are hydrogen or fluoro and $X_3$–$X_6$ have the meanings given above, and

Y is oxygen, sulphur or imino,

Q is $(CR_6R_7)_p$, in which p is 0 or 1,

$R_2$ is hydrogen, alkyl of 1–6 C-atoms, optionally substituted by an hydroxy or amino group, or is $C_1$–$C_4$-alkoxycarbonyl, cyano or di-$C_{1-C4}$-alkylaminocarbonyl,

$R_3$, $R_4$, $R_5$, $R_6$, and $R_7$ are hydrogen or methyl, or $R_3$ and $R_4$ together are trimethylene, tetramethylene or 1,3-butadienylene when $R_2$ and $R_5$ together are a direct bond.

2. (5Z,13E)-(8R,9S,11R,12R,15S)-2-(2-Oxazolin-2-yl)-1-nor-5,13-prostadiene-9,11,15-triol.

3. 2-[4-(E)-(1S,5S,6R,7R)-7-Hydroxy-6-[(E)-(3S,4RS)-3-hydroxy-4-methyloct-1-en-6-inyl]bicyclo[3.3.0]octan-3-ylidene)-butyl]-2-oxazoline.

4. 2-{(E)-(1S,5R,6R)-7-Hydroxy-6-[(E)-(3S,4RS)-3-hydroxy-4-methyl-1-octenyl]-2-oxabicyclo[3.3.0]octan-3-yliden}-5-(2-oxazolin-2-yl)pentanenitrile.

5. Process for the preparation of compounds of general formula I, characterised in that compounds of general formula II

$$R_1–COOR_{12} \qquad (II)$$

wherein $R_1$ has the meaning given above and $R_{12}$ is hydrogen, trialkylsilyl or $C_1$–$C_6$-alkyl are reacted with an amine of general formula III

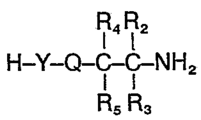
III,

wherein $R_2$, $R_3$, $R_4$, $R_5$, Q and Y have the meanings given above, optionally after protection of any free hydroxy groups present, with the assistance of organic phosphines or phosphonium salts and perhalogenated hydrocarbons or ketones in the presence of tertiary bases, and optionally isomers are separated and/or protected hydroxy groups are freed and/or free hydroxy groups are esterified or etherified and/or hydroxy groups are oxidised and/or oxo groups are reduced and/or double or triple bonds are hydrogenated.

6. Pharmaceutical compositions comprising one or more compounds according to claim 1 together with conventional adjuvants and carriers.

**Revendications**

1. Prostaglandines répondant à la formule I:

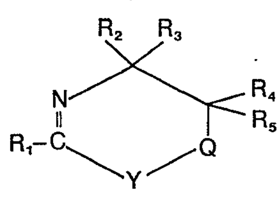
I,

dans laquelle:

$R_1$ représente un radical de prostaglandine de formule:

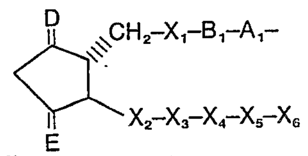
,

dans laquelle:

$A_1$ représente un radical $-(CH_2)_n-$ ou $-CF_2-(CH_2)_m-$ (l'indice n étant un nombre de 1 à 3 et l'indice m un nombre égal à 1 ou 2) ou un radical $-CH=CH-CH_2-$ ou $-CH_2-CH=CH-$, et

$B_1$–$X_1$ représente $-(CH_2)_3-$,

$A_1$–$B_1$–$X_1$ représente $-(CH_2)_5-$,

$A_1$–$B_1$ représente $-(CH_2)_o-$, $-CF_2-(CH_2)_n-$, $-CH=CH-(CH_{2m}-$, $-(CH_2)_m-CH=C-$, l'indice m étant un nombre égal à 1 ou à 2, l'indice n un nombre de 1 à 3 et l'indice o un nombre de 1 à 4, et

$X_1$ représente un radical alcénylène

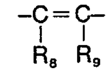

cis dans lequel les symboles $R_8$ et $R_9$ représentent chacun l'hydrogène ou un alkyle en $C_1$–$C_6$, ou

$A_1$ représente $-CH_2-$,

$B_1$ représente l'oxygène ou $-CH_2-$ et

D représente un atome d'oxygène, à la fois un atome d'hydrogène et un radical hydroxy ayant la configuration α ou la configuration β, à la fois un atome d'hydrogène et un atome d'halogène ayant la configuration α ou la configuration β, ou un radical $CH_2$,

E représente un atome d'oxygène, à la fois un atome d'hydrogène et un radical hydroxy ayant la configuration α, à la fois un atome d'hydrogène et un radical α-$CH_3$, ou à la fois un atome d'hydrogène et un radical α-$CH_2OH$,

$X_2$ représente $-CH_2-CH_2-$, $-C\equiv-$ ou un radical alcénylène

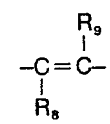

trans dans lequel les symboles $R_8$ et $R_9$ ont les significations précédemment données,

$X_3$ représente

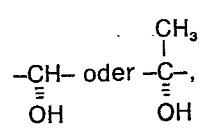
oder

$X_4$ représente un radical $-(CH_2)_n-$ dans lequel n est un nombre de 1 à 3, ou un radical

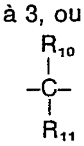

dans lequel $R_{10}$ et $R_{11}$ représentent chacun un atome d'hydrogène ou de fluor ou un radical méthyle ou méthoxy ou forment ensemble un radical $-CH_2-CH_2-$ ou $-CH_2-CH_2-CH_2-$,

$X_5$ représente $-CH_2-$, l'oxygène, le soufre ou une liaison directe, et

$X_6$ représente un radical phényle, chloro-3 phényle, chloro-4 phényle ou trifluorométhyl-3 phényle, ou, dans le cas où $X_5$ est une liaison directe, $X_6$ représente l'un des radicaux:

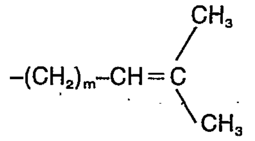,

$-(CH_2)_m-C\equiv C-CH_3$ et $-(CH_2)_m-C\equiv C-CH_2-CH_3$ dans lesquels m est un nombre égal à 1 ou à 2, ou l'un des radicaux $-(CH_2)_4-OCH_3$, $-CH(CH_3)-CH_2-CH_2-CH_3$ et $-(CH_2)_3-CH=CH_2$, ou

$R_1$ représente un radical de prostacycline répondant à la formule:

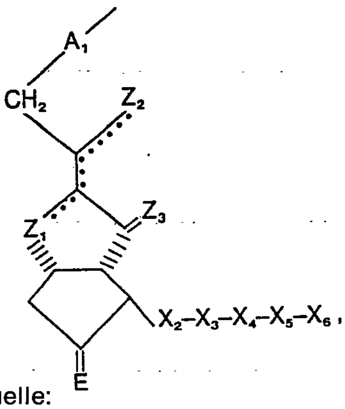,

dans laquelle:

$A_1$, E et $X_2$ à $X_6$ ont les significations qui ont été données plus haut,

$Z_1$ représente l'oxygène ou $CH_2$ dans le cas où $Z_2$ désigne l'hydrogène ou un radical cyano, et l'azote dans le cas où $Z_2$ désigne deux atomes d'hydrogène ($H_2$), et

$Z_3$ représente $H_2$ ou l'oxygène, ou

$R_1$ représente un radical de prostacycline répondant à la formule:

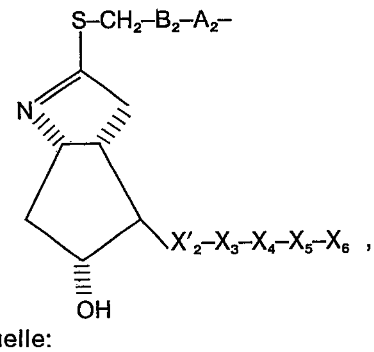,

dans laquelle:

$A_2$ représente $-CH_2-$, $-CF_2-$, $-\underset{CH_3}{\underset{|}{CH}}-$ ou, dans le cas où $B_2$ désigne une liaison simple, un radical alcénylène

$$-\underset{R_8}{\underset{|}{C}}=\overset{R_9}{\overset{|}{C}}-$$

trans,

$B_2$ représente $-CH_2-$ ou une liaison simple,

$X'_2$ représente $-CH_2-CH_2-$, $-C\equiv C-$ ou un radical alcénylène

$$-\underset{R_8}{\underset{|}{C}}=\overset{R_9}{\overset{|}{C}}-$$

trans dans lequel $R_8$ et $R_9$ représentent chacun l'hydrogène ou le fluor, et

$X_3$ à $X_6$ ont les significations précédemment données,

Y représente l'oxygène, le soufre ou un radical imino,

Q représente un radical $(CR_6R_7)_p$ dont l'indice p est égal à 0 ou à 1,

$R_2$ représente l'hydrogène, un alkyle en $C_1$–$C_6$ éventuellement porteur d'un radical hydroxy ou amino, un radical alcoxycarbonyle dont l'alcoxy contient de 1 à 4 atomes de carbone, un radical cyano ou un radical dialkylamino-carbonyle dont chacun des alkyles contient de 1 à 4 atomes de carbone,

et

$R_3$, $R_4$, $R_5$, $R_6$ et $R_7$ représentent chacun l'hydrogène ou un méthyle, $R_3$ et $R_4$ pouvant également former ensemble un radical triméthylène, tétraméthylène ou butadiène-1,3-ylène dans lequel $R_2$ et $R_5$ forment ensemble une liaison directe.

2. ($\Delta^2$-Oxazolinyl-2)-2 nor-1 prostadiène-5,13 triol-9,11,15 (5Z,13E)-(8R,9S,11R,12R,15S).

3. {[Hydroxy-7 [hydroxy-3 méthyl-4 octène-1 yne-6-yl (E)-(3S,4RS)]-6 bicyclo[3.3.0]octylidène-3 (E)-(1S,5S,6R,7R]-4 butyl}-2 oxazoline.

4. {Hydroxy-7 [hydroxy-3 méthyl-4 octène-1 yl (E)-(3S,4RS)]-6 oxa-2 bicyclo[3.3.0] octylidène-3 (E)-(1S,5R,6R)}-2 ($\Delta^2$-oxazolinyl-2)-5 pentane-nitrile.

5. Procédé de préparation de composés répondant à la formule générale I, procédé caractérisé en ce qu'on fait réagir, éventuellement après en avoir protégé les radicaux hydroxy libres, des composés répondant à la formule générale II:

$$R_1-COOR_{12} \qquad II$$

dans laquelle $R_1$ a la signification indiquée cidessus et $R_{12}$ représente l'hydrogène, un radical trialkylsilyle ou un radical alkyle en $C_1$–$C_6$, avec une amine répondant à la formule générale III:

$$H-Y-Q-\underset{R_5}{\underset{|}{\overset{R_4}{\overset{|}{C}}}}-\underset{R_3}{\underset{|}{\overset{R_2}{\overset{|}{C}}}}-NH_2 \qquad III,$$

dans laquelle $R_2$, $R_3$, $R_4$, $R_5$, Q et Y ont les significations précédemment données, au moyen de phosphines organiques ou de sels de phosphoniums organiques et d'hydrocarbures perhalogénés ou de cétones, en présence de bases tertiaires, après quoi, le cas échéant, on sépare les isomères et/ou on libère des radicaux hydroxy protégés et/ou on estérifie ou éthérifie des radicaux hydroxy libres et/ou on oxyde des radicaux hydroxy et/ou on réduit des radicaux oxo et/ou on hydrogène des liaisons doubles ou triples.

6. Médicament constitué d'un ou plusieurs composés selon la revendication 1 ainsi que d'adjuvants et d'excipients usuels.